# FASCICULE DE BREVET EUROPEEN

(11) **EP 2 477 554 B1**
(45) Date de publication et mention de la délivrance du brevet: **28.08.2013**
(21) Numéro de dépôt: 10752838.2
(22) Date de dépôt: 16.09.2010
(51) Int. Cl.: A61B 10/02, A61B 17/00, A61B 17/34, A61B 19/00

(54) **INSTRUMENT CHIRURGICAL DE PRELEVEMENT MOLECULAIRE**
CHIRURGISCHES INSTRUMENT FÜR MOLEKULARE PROBENNAHME
SURGICAL INSTRUMENT FOR MOLECULAR SAMPLING

(30) Priorité: 18.09.2009 FR 0956419
(43) Date de publication de la demande: 25.07.2012
(73) Titulaire: Universite Joseph Fourier - Grenoble 1, 38400 St. Martin d'Heres (FR); Commissariat à l'Énergie Atomique et aux Énergies Alternatives, 75015 Paris (FR); Centre Hospitalier Universitaire Grenoble, 38700 La Tronche (FR)
(72) Inventeur: MONTESSUY, Renaud, F-38320 Eybens (FR); RATEL, David, F-07130 Saint-peray (FR); GAY, Emmanuel, F-38700 Corenc (FR); PASSAGIA, Jean-Guy, F-38410 Vaulnaveys Le Haut (FR); BERGER, François, F-38240 Meylan (FR); ISSARTEL, Jean-Paul, F-38120 Saint Egreve (FR)
(74) Mandataire: Regimbeau
(86) Numéro de dépôt international: PCT/EP2010/063587
(87) Numéro de publication internationale: WO 2011/033008

(56) Documents cités:
- EP-A2- 1 724 579
- WO-A1-2006/082344
- WO-A1-2010/025719
- WO-A2-2006/090220
- WO-A2-2008/110392

## Description

### DOMAINE DE L'INVENTION

La présente invention concerne un instrument chirurgical de prélèvement moléculaire, comprenant un guide et un stylet supportant un dispositif de capture de molécules.

### ARRIERE PLAN DE L'INVENTION

La capture de bio-marqueurs dans le corps d'un patient est utile pour le diagnostic et la localisation de lésions, telles que des tumeurs.

Les bio-marqueurs sont des molécules, par exemple des protéines, qui sont spécifiques de la lésion considérée.

Ainsi, par exemple, la protéine ACTH est un bio-marqueur de l'adénome corticotrope hypophysaire, ou maladie de Cushing.

Avant ou pendant une intervention chirurgicale sur la lésion, il est généralement nécessaire de repérer précisément les contours de cette lésion, dans le but de pouvoir la retirer en totalité sans retirer de tissus sains.

La capture de bio-marqueurs en pré- et/ou per-opératoire fait actuellement l'objet d'expérimentations en vue de certaines interventions.

On peut citer par exemple une biopsie moléculaire intracrânienne par stéréotaxie pour le diagnostic de gliomes.

Cette intervention consiste à insérer dans la tête du patient, à travers une grille stéréotaxique, un stylet muni à son extrémité distale d'une plaquette de silicium activée pour capturer la ou les molécule(s) recherchée(s).

Cette technique supposant de traverser plusieurs tissus, il est nécessaire de protéger la plaquette de silicium de toute contamination en-dehors de la lésion elle-même.

Il a donc été proposé, dans le document WO 2006/090220, de procurer un guide renfermant le stylet en autorisant le coulissement et la rotation du stylet à l'intérieur du guide.

Le guide présente à son extrémité distale une fenêtre qui consiste en une ouverture pratiquée dans sa paroi.

Pendant l'insertion dans le crâne, le stylet est positionné dans le guide de sorte que la face activée de la plaquette de silicium soit opposée à la fenêtre, de manière à être protégée.

Lorsque l'extrémité du guide a atteint sa cible, le chirurgien tourne le stylet d'environ 180° pour que la face activée de la plaquette de silicium soit exposée.

La durée nécessaire pour la capture des molécules étant atteinte, le chirurgien tourne à nouveau le stylet d'environ 180° dans le guide, de sorte que la face activée de la plaquette de silicium soit à nouveau protégée lors du trajet de retour du stylet.

Toutefois, cet instrument présente des limitations notamment en termes d'encombrement et d'utilisation.

En effet, la nécessité de disposer un guide autour du stylet augmente l'encombrement de l'instrument. Le diamètre extérieur de l'instrument inséré dans les tissus est ainsi de l'ordre de 2 à 5 mm.

Par ailleurs, il n'est pas utilisable dans le traitement de certaines pathologies, notamment pour la détection de l'adénome hypophysaire (ou maladie de Cushing).

En effet, la glande hypophysaire se situe à un emplacement tel qu'il faudrait perforer plusieurs organes particulièrement sensibles pour pouvoir y accéder par cette voie.

En outre, comme on cherche à capturer la protéine ACTH afin de localiser plus précisément la lésion, en vue d'une exérèse dans le même temps opératoire, l'approche stéréotaxique décrite dans le document précité ne présente que peu d'intérêt.

A l'heure actuelle" il est connu également de réaliser une ou plusieurs biopsies extemporanées, en prélevant des tissus dans des régions d'intérêt de l'organe considéré en vue d'une analyse pendant le temps opératoire.

Toutefois, cette technique est agressive.

On cherche au contraire à mettre en oeuvre des procédures minimalement invasives.

Un but de la présente invention est donc de procurer un instrument de capture moléculaire minimalement invasif permettant de remplacer la biopsie par voie stéréotaxique lorsque celle-ci n'est pas envisageable, et notamment adaptée à des prélèvements intra-hypophysaires.

Un autre but de l'invention est de procurer un instrument dont l'encombrement est plus faible que dans l'art antérieur.

### BREVE DESCRIPTION DE L'INVENTION

Conformément à l'invention, il est proposé un instrument chirurgical pour le prélèvement de molécules dans un organe dont l'accès est possible directement ou par une voie d'abord chirurgicale, comprenant :
- un dispositif de capture desdites molécules,
- un stylet supportant ledit dispositif de capture et apte à pénétrer dans l'organe,
- un guide pour l'insertion du stylet dans le corps jusqu'à l'organe,
ledit instrument étant caractérisé en ce que l'extrémité distale du guide est munie d'un patin d'amortissement destiné à venir en appui contre l'organe.

Dans le présent texte, on entend par « extrémité distale » l'extrémité de l'instrument qui est insérée dans le corps du patient, vers l'organe ciblé ; et par « extrémité proximale » l'extrémité manipulée par le chirurgien.

Par « organe accessible directement » on entend dans le présent texte tout organe dans lequel on peut capturer des bio-marqueurs sans traverser au préalable d'autres organes.

L'invention concerne également le prélèvement d'organes dont l'accès est possible au moyen d'une voie d'abord réalisée préalablement, c'est-à-dire qu'une fois la voie d'abord réalisée, l'instrument n'a pas de tissus à traverser pour atteindre l'organe.

Selon d'autres caractéristiques avantageuses de l'invention :
- l'extrémité distale du stylet est également munie d'un patin d'amortissement disposé de sorte à assurer une étanchéité vis-à-vis du guide ;
- le patin d'amortissement est réalisé en un matériau polymère biocompatible non protéique ;
- le patin d'amortissement présente vis-à-vis de l'organe un coefficient de frottement supérieur à celui de l'acier ;
- le dispositif de capture des molécules est disposé dans un logement de la portion distale du stylet, de sorte que seule sa face active soit exposée ;
- le dispositif de capture est adapté pour capturer des protéines, mais aussi, le cas échéant, tout autre matériel biologique ;
- le stylet et/ou le guide est muni d'un capteur de neuro-navigation ;
- l'instrument comporte une butée réglable pour contrôler la profondeur d'insertion du stylet dans l'organe ;
- l'instrument comporte plusieurs guides parallèles pour un prélèvement simultané de molécules en différents endroits de l'organe, l'extrémité distale de chaque guide étant munie d'un patin d'amortissement individuel ou commun à l'ensemble des guides.

### BREVE DESCRIPTION DES DESSINS

D'autres caractéristiques et avantages de l'invention ressortiront de la description détaillée qui va suivre, en référence aux dessins annexés sur lesquels :
- la figure 1 est une vue d'ensemble de l'instrument conforme à l'invention, en situation de prélèvement,
- la figure 2 est une vue de dessus du stylet,
- la figure 3 est une vue de profil du stylet,
- la figure 4 est une section (grossie) du stylet selon la coupe A-A représentée à la figure 3,
- la figure 5 est une vue de gauche (grossie) du stylet illustré à la figure 3,
- les figures 6A et 6B illustrent deux modes de mise en oeuvre de patins d'amortissement,
- la figure 7 est une vue de détail de l'extrémité distale du guide et du stylet en situation de prélèvement.

### DESCRIPTION DETAILLEE DE L'INVENTION

La présente invention va maintenant être décrite en détail, mais de manière non limitative, en référence à la capture de bio-marqueurs dans la glande hypophysaire, notamment pour repérer la protéine ACTH (hormone adénocorticotrope), qui est un marqueur spécifique de l'adénome corticotrope hypophysaire, ou maladie de Cushing.

La glande hypophysaire est située directement à l'arrière du sphénoïde.

L'adénome hypophysaire s'opère donc par voie trans-sphénoïdale, ce qui implique un passage par les fosses nasales, dans une voie d'abord étroite et profonde, avant de déboucher dans le sinus sphénoïdal, donnant accès à la loge hypophysaire.

Cependant, la glande hypophysaire est accessible directement, c'est-à-dire qu'il n'est pas nécessaire de traverser de tissus pour l'atteindre une fois la voie d'abord réalisée.

Il en résulte que, contrairement au dispositif de capture par stéréotaxie mentionné en préambule, le dispositif de capture présente un moindre risque d'être contaminé pendant la traversée du corps du patient, avant d'atteindre la glande hypophysaire.

On peut donc s'affranchir du dispositif à fenêtre et proposer un dispositif pénétrant l'organe moins encombrant que celui de l'art antérieur.

Toutefois, compte tenu des importants saignements générés pendant cette intervention, il est nécessaire d'assurer l'étanchéité du dispositif de capture vis-à-vis de ces saignements.

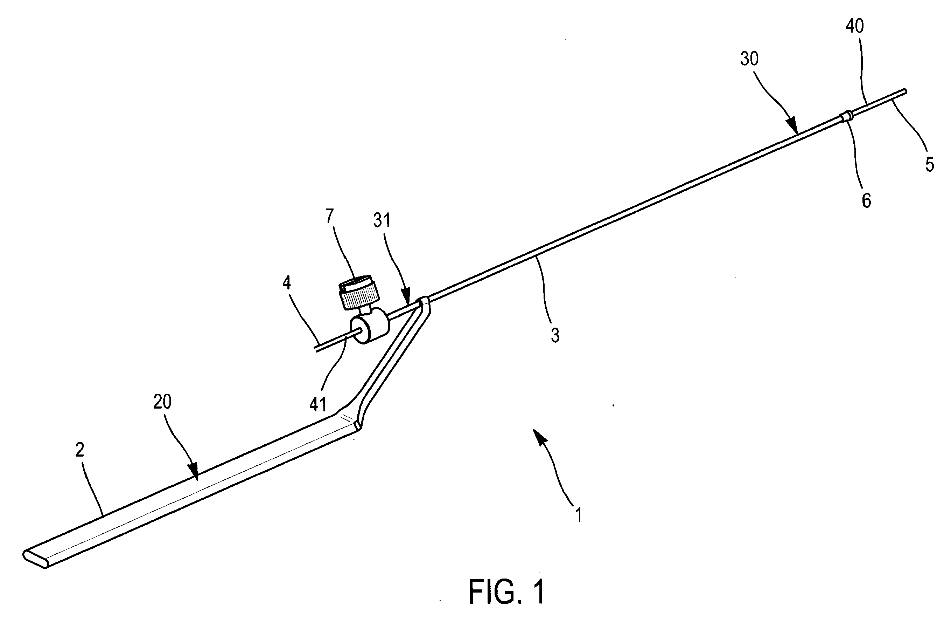

La figure 1 est une vue d'ensemble de l'instrument.

Comme on peut le voir, l'instrument 1 comprend un support 2 adapté pour la préhension par le chirurgien, le support 2 étant prolongé par un guide 3 dans lequel est monté en coulissement un stylet 4 portant à son extrémité distale un dispositif de capture 5.

### Guide et support

Le guide 3 se présente sous la forme générale d'un tube et possède une portion distale 30 destinée à être insérée dans le corps du patient.

Le diamètre intérieur du guide 3 est adapté pour l'insertion et le coulissement du stylet 4.

Typiquement, le diamètre intérieur du guide est de 900 µm, le diamètre extérieur du stylet étant de l'ordre de 800 µm.

Dans sa portion proximale 31, le guide 3 est relié au support 2.

Par exemple, le guide 3 et le support 2 sont tous deux métalliques et le guide 3 est inséré dans un alésage prévu à cet effet sur le support 2 puis soudé par laser.

Bien sûr, tout autre mode d'assemblage du guide 3 et du support 2 - voire leur réalisation en une seule pièce ou au contraire démontable (pour permettre l'utilisation de plusieurs guides lors de l'intervention, par exemple lorsqu'un guide est contaminé) - est envisageable.

Avantageusement, l'ensemble guide 3/support 2 a une forme de baïonnette, c'est-à-dire que la zone de préhension 20 et le guide 3 présentent des axes sensiblement parallèles mais décalés de quelques centimètres l'un par rapport à l'autre.

Ceci permet que la vision de l'extrémité distale 30 du guide 3 ne soit pas entravée par la main du chirurgien qui manipule l'instrument 1.

### Stylet

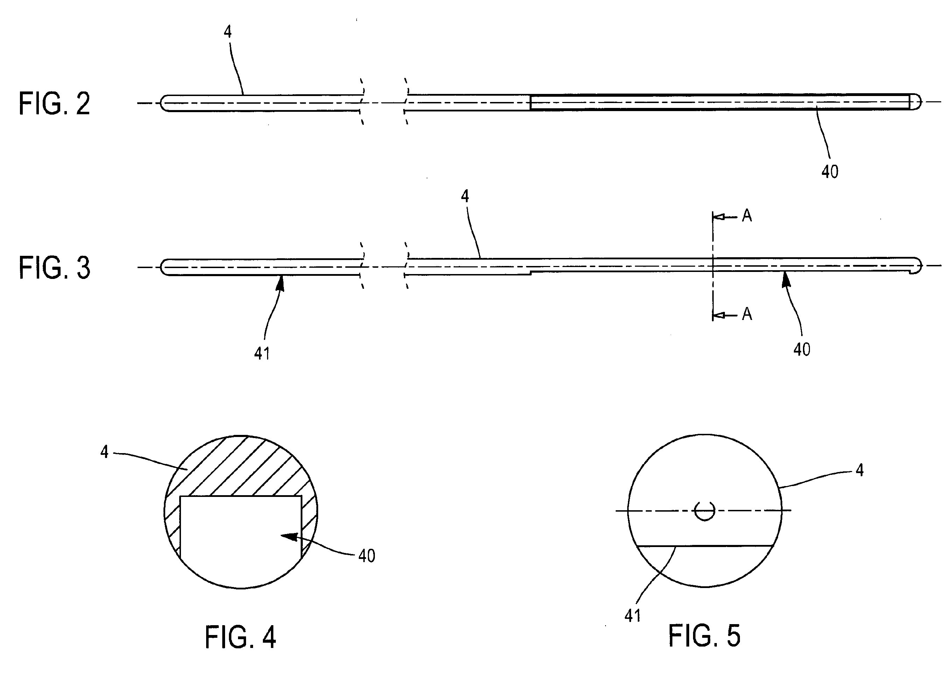

En référence aux figures 2 à 5, le stylet 4 présente une forme généralement cylindrique, sauf à son extrémité distale où il comporte un logement 40 pour le dispositif de capture moléculaire, et à son extrémité proximale où il comporte un méplat 41 de repérage.

L'instrument 1 est pourvu, dans sa portion proximale, d'une butée réglable 7 qui permet de limiter la profondeur d'insertion du stylet 4 par rapport à l'extrémité distale du guide 3.

Tant le stylet 4 que le guide 3 sont réalisés dans des matériaux biocompatibles, comme par exemple l'acier inoxydable chirurgical, ou tout autre métal biocompatible, ferromagnétique ou non.

### Dispositif de capture

Le dispositif de capture 5 se présente sous la forme générale d'une plaquette comprenant une surface comportant des groupements chimiques activés pour la capture des molécules recherchées.

Par exemple, on pourra utiliser des puces de silicium telles que décrites dans les documents WO 2006/082344 et « A new micro minimally invasive biopsy tool for molecular analysis », Cosnier et al., Proceedings of the 28th IEEE, EMBS Annual Conference, New York City, USA, Aug 30-Sept 3, 2006, mais tout autre support adapté pour l'adsorption des molécules considérées peut être utilisé.

Les dimensions du dispositif de capture sont typiquement : une longueur de 20 mm, une largeur de 650 µm et une épaisseur de 300 µm.

Naturellement, on pourra utiliser un dispositif de capture présentant une forme autre que rectangulaire, et présentant d'autres dimensions.

Par ailleurs, le dispositif de capture peut être scindé en différentes zones, chacune pouvant donner une indication pour différentes profondeurs de l'organe

La plaquette de silicium présentant des bords tranchants, et étant de ce fait susceptible d'agresser les parois des cavités traversées, elle est insérée dans un logement 40 ménagé à cet effet dans le stylet 4.

Le dispositif de capture 5 est fixé de manière amovible sur le stylet 4 par tout moyen approprié tel que collage, clipsage, vissage, etc.

Le logement 40 est mieux visible sur la figure 4, où l'on peut constater qu'il est constitué par un évidement de forme rectangulaire dans la section du stylet 4.

Le logement 40 présente des dimensions sensiblement égales aux dimensions du dispositif de capture, ou légèrement supérieures à celles-ci pour faciliter le montage et le démontage du dispositif de capture.

Les bords du dispositif de capture (non illustré à la figure 4) sont donc protégés par les parois du logement 40.

Le logement 40 présente l'avantage supplémentaire de protéger le dispositif de capture 5 contre une désolidarisation intempestive vis-à-vis du stylet alors que l'instrument est à l'intérieur du corps du patient.

Le logement 40 et donc le dispositif de capture 5 sont situés dans la position la plus distale possible du stylet 4 (c'est-à-dire, à une distance typiquement de 400 à 600 µm par rapport à l'extrémité distale du stylet), afin d'offrir la plus grande surface de contact possible avec l'organe ciblé.

En référence à la figure 5, le stylet 4 comporte, à son extrémité proximale, un méplat 41 servant au repérage de la position du logement 40.

Ce méplat 41 est par exemple réalisé à une distance de 200 µm de l'axe du stylet.

### Patin d'amortissement

L'extrémité distale du guide 3 est munie d'un patin d'amortissement 6 destiné à venir en appui contre l'organe sans le pénétrer.

La surface de contact du patin d'amortissement 6 contre l'organe est typiquement une couronne présentant un diamètre inférieur généralement inférieur à 2,5 mm, le patin 6 devant permettre le passage de l'extrémité distale du stylet 4 en vue de la capture des molécules.

De manière particulièrement avantageuse, le patin d'amortissement 6 est réalisé dans un matériau polymère non protéique et biocompatible, par exemple du silicone.

Le patin 6 est réalisé en un matériau suffisamment souple et déformable et présente à son extrémité distale une surface suffisante pour assurer le contact avec l'organe et pour amortir tout choc lorsque le guide 3 arrive en butée contre l'organe.

Le patin 6 est également conçu de sorte à ne pas présenter d'arêtes tranchantes.

Il présente aussi de préférence des propriétés antidérapantes, permettant ainsi de limiter tout déplacement relatif de l'instrument par rapport à l'organe au cours du prélèvement. Ainsi, le coefficient de frottement du patin 6 sur l'organe est supérieur au coefficient de frottement de l'acier sur l'organe.

Enfin, le patin d'amortissement 6 assure une fonction d'étanchéité vis-à-vis d'une éventuelle contamination sanguine.

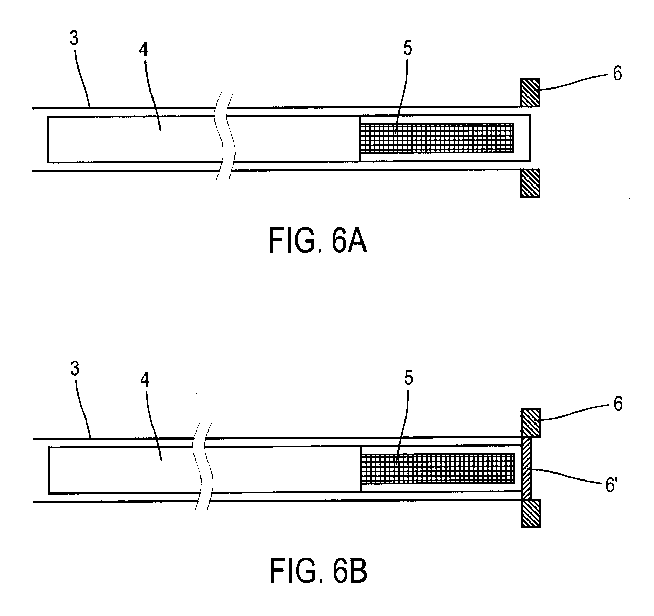

Selon un premier mode de réalisation (illustré de manière schématique à la figure 6A), seule l'extrémité distale du guide 3 est munie dudit patin 6.

Selon une variante de réalisation (illustrée schématiquement à la figure 6B), l'extrémité distale du stylet 4 est elle aussi coiffée d'un patin 6' similaire au patin 6.

De préférence, le patin 6' est profilé, par exemple en forme d'ogive, afin de faciliter l'insertion du stylet dans l'organe.

Ceci permet de mieux protéger le dispositif de capture 5 vis-à-vis des saignements ambiants.

Le patin 6, le cas échéant 6', est fixé sur le guide 3, respectivement sur le stylet 4, de manière à éviter toute désolidarisation intempestive lorsque ceux-ci sont à l'intérieur du corps du patient.

Selon un mode de réalisation particulier, on emmanche un patin de silicone autour de la pièce (guide ou stylet) métallique.

On pourra aussi par exemple employer une colle présentant une adhérence appropriée vis-à-vis du matériau du patin et de celui du guide (et, le cas échéant, du stylet).

### Instrument multi-prélèvements

Selon une variante de réalisation (non illustrée), le support peut supporter plusieurs guides parallèles destinés à être insérés simultanément jusqu'à l'organe, chaque guide étant muni d'un patin d'amortissement tel que décrit plus haut.

A titre alternatif, au lieu de patins d'amortissement individuels disposés à l'extrémité distale de chaque guide, un unique patin de forme appropriée peut être disposé à l'extrémité distale de l'ensemble des guides de sorte à être commun à ceux-ci.

On introduit alors un stylet dans chacun des guides, ce qui permet de faire un prélèvement simultané en plusieurs endroits de l'organe, d'où un gain de temps notable sur l'intervention.

### Neuro-navigation

Bien que l'organe ciblé soit visible et accessible par le chirurgien sans imagerie médicale, il peut être avantageux de localiser le dispositif de capture sur une image sur laquelle la lésion est également visible.

Ceci permet en effet de définir plus précisément la trajectoire du guide et du stylet, en vue de cibler plus précisément la zone de prélèvement.

A cet effet, on peut équiper le stylet et/ou le guide d'un capteur permettant de déterminer la position du stylet dans le repère de l'image.

Par exemple, des diodes infra rouges peuvent être assemblées sur le guide, de même qu'une diode démontable (constituant un système équivalent à la butée) sur le stylet.

Le déplacement relatif de cette diode par rapport aux autres permet un repérage de profondeur, alors que les diodes du guide fournissent une information de positionnement et de trajectoire par le biais d'une camera idoine, transmettant ces infos au système de neuro-navigation.

### Utilisation de l'instrument

Lors d'une intervention chirurgicale visant à capturer la protéine ACTH, bio-marqueur de l'adénome hypophysaire, l'instrument 1 est utilisé de la manière suivante.

Le chirurgien introduit tout d'abord le guide 3 à l'intérieur de la cavité nasale puis du sphénoïde du patient, jusqu'à atteindre la glande hypophysaire.

Grâce à la forme en baïonnette du support 2, le champ visuel du chirurgien est libre et il peut voir directement la glande hypophysaire.

De manière alternative, lorsque le guide 3 et/ou le stylet 4 sont visibles dans un outil de neuro-navigation, le chirurgien vérifie la trajectoire du guide sur l'image.

Une fois le guide 3 arrivé en butée contre la glande par l'intermédiaire du patin d'amortissement 6, le chirurgien introduit dans celui-ci le stylet 4 pourvu du dispositif de capture 5 à son extrémité distale.

Dans ce cas, le stylet peut être accompagné d'un mandrin dans le guide.

Il est également possible d'introduire le guide dans l'organe du patient alors que le stylet est déjà inséré dans le guide, notamment lorsque le stylet est muni d'un patin d'amortissement, ou en cas de distance faible entre le microscope et l'instrument.

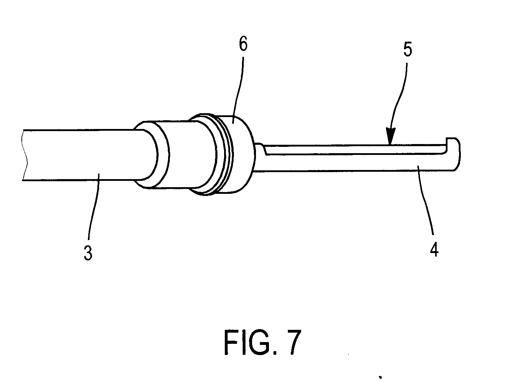

L'extrémité distale du stylet 4 débouche alors de l'extrémité distale du guide 3 et perfore la glande jusqu'à arriver à la butée 7, mettant ainsi en contact le dispositif de capture 5 avec le tissu de la glande, et permettant la capture de la protéine ACTH. Cette situation est illustrée à la figure 7.

De ce fait, seule l'extrémité distale du stylet comportant le dispositif de capture de l'instrument, qui présente un diamètre maximum de 800 µm, est insérée dans le tissu.

La butée 7 permet d'éviter que le stylet 4 ne soit inséré trop profondément dans ou à travers la glande hypophysaire, qui est de petites dimensions (de l'ordre de 1 cm chez un sujet adulte).

Après une durée suffisante pour capturer une quantité significative de molécules (typiquement, 10 à 30 secondes), le chirurgien retire le stylet et le guide, ou bien décale légèrement le guide et insère un nouveau stylet pour effectuer un prélèvement dans une zone différente de la glande hypophysaire.

Une fois le(s) prélèvement(s) effectué(s), le dispositif de capture 5 est alors démonté du stylet 4 par tout moyen adéquat selon le mode de fixation employé, en vue de la détection de la protéine ACTH.

On comprend donc que l'instrument qui vient d'être décrit présente l'avantage de minimiser l'agressivité des techniques chirurgicales intra hypophysaires en réalisant l'exérèse de manière plus précise.

Ceci permet de limiter les insuffisances endocriniennes post-opératoires et la morbidité chirurgicale.

### Détection des molécules capturées

Une fois le dispositif de capture 5 démonté du stylet 4, la détection des molécules peut être effectuée par différentes techniques.

Selon une première possibilité, on procède à une élution des bio-marqueurs adsorbés et on mesure la quantité de ces molécules dans la solution obtenue.

Une autre technique, particulièrement avantageuse, consiste à placer le dispositif de capture dans un système de spectrométrie de masse, tel que celui qui est décrit par exemple dans le document WO 2006/090220.

L'avantage de cette technique est de permettre une lecture directe de la quantité de molécules adsorbées.

Bien que l'invention ait été principalement décrite pour une application à la capture de bio-marqueurs d'une tumeur hypophysaire, elle s'applique également à la capture de molécules dans tout autre organe accessible directement.

La présente invention trouve donc application dans la capture de bio-marqueurs de tumeurs ORL (oto-rhino-laryngologiques), de la peau, de muscles ou encore de tumeurs gynécologiques.

Elle présente également un intérêt en pathologie infectieuse (diagnostic des abcès par exemple).

## Revendications

1. Instrument chirurgical (1) pour le prélèvement de molécules dans un organe dont l'accès est possible directement ou par une voie d'abord chirurgicale, comprenant :
- un dispositif (5) de capture desdites molécules,
- un stylet (4) supportant ledit dispositif de capture (5) apte à pénétrer dans l'organe,
- un guide (3) pour l'insertion du stylet (4) dans le corps jusqu'à l'organe,
**caractérisé en ce que** l'extrémité distale du guide (3) est munie d'un patin d'amortissement (6) destiné à venir en appui contre l'organe.

2. Instrument selon la revendication 1, **caractérisé en ce que** l'extrémité distale du stylet (4) est également munie d'un patin d'amortissement (6') disposé de sorte à assurer une étanchéité vis-à-vis du guide (3).

3. Instrument selon l'une des revendications 1 ou 2, **caractérisé en ce que** le patin d'amortissement (6, 6') est réalisé en un matériau polymère biocompatible non protéique.

4. Instrument selon l'une des revendications 1 à 3, **caractérisé en ce que** le patin d'amortissement (6, 6') présente vis-à-vis de l'organe un coefficient de frottement supérieur à celui de l'acier.

5. Instrument selon l'une des revendications 1 à 4, **caractérisé en ce que** le dispositif de capture (5) est disposé dans un logement (40) de la portion distale du stylet (4), de sorte que seule sa face active soit exposée.

6. Instrument selon l'une des revendications 1 à 5, **caractérisé en ce que** le dispositif de capture (5) est adapté pour capturer des protéines ou toute autre biomolécule.

7. Instrument selon l'une des revendications 1 à 6, **caractérisé en ce que** le stylet (4) et/ou le guide (3) est muni d'un capteur de neuro-navigation.

8. Instrument selon l'une des revendications 1 à 7, **caractérisé en ce qu'**il comporte une butée réglable (7) pour contrôler la profondeur d'insertion du stylet (4) dans l'organe.

9. Instrument selon l'une des revendications 1 à 8, **caractérisé en ce qu'**il comprend plusieurs guides parallèles pour un prélèvement simultané de molécules en différents endroits de l'organe.

## Patentansprüche

1. Chirurgisches Instrument (1) für die Entnahme von Molekülen aus einem Organ, zu dem der Zugang direkt oder über einen chirurgischen Zugang möglich ist, umfassend:
- eine Vorrichtung (5) zum Fangen der Moleküle,
- ein Stilett (4), das die Fangvorrichtung (5) trägt und in das Organ eindringen kann,
- eine Führung (3) für das Einführen des Stiletts (4) in den Körper bis zu dem Organ,
**dadurch gekennzeichnet, dass** das distale Ende der Führung (3) mit einem Dämpfungsfuß (6) versehen ist, der dafür bestimmt ist, gegen das Organ zum Aufliegen zu kommen.

2. Instrument nach Anspruch 1, **dadurch gekennzeichnet, dass** das distale Ende des Stiletts (4) auch mit einem Dämpfungsfuß (6') versehen ist, der derart angeordnet ist, dass er eine Abdichtung gegenüber der Führung (3) bewirkt.

3. Instrument nach einem der Ansprüche 1 oder 2, **dadurch gekennzeichnet, dass** der Dämpfungsfuß (6, 6') aus einem proteinfreien bioverträglichen Polymermaterial realisiert ist.

4. Instrument nach einem der Ansprüche 1 bis 3, **dadurch gekennzeichnet, dass** der Dämpfungsfuß (6, 6') gegenüber dem Organ einen Reibungskoeffizienten aufweist, der höher als der von Stahl ist.

5. Instrument nach einem der Ansprüche 1 bis 4, **dadurch gekennzeichnet, dass** die Fangvorrichtung (5) in einer Aufnahme (40) des distalen Abschnitts des Stiletts (4) anordnet ist, derart, dass nur ihre aktive Fläche exponiert ist.

6. Instrument nach einem der Ansprüche 1 bis 5, **dadurch gekennzeichnet, dass** die Fangvorrichtung (5) dafür geeignet ist, Proteine oder jedes andere Biomolekül zu fangen.

7. Instrument nach einem der Ansprüche 1 bis 6, **dadurch gekennzeichnet, dass** das Stilett (4) und/oder die Führung (3) mit einem Neuronavigations-Sensor versehen ist.

8. Instrument nach einem der Ansprüche 1 bis 7, **dadurch gekennzeichnet, dass** es einen einstellbaren Anschlag (7) aufweist, um die Eindringtiefe des Stilettes (4) in das Organ zu steuern.

9. Instrument nach einem der Ansprüche 1 bis 8, **dadurch gekennzeichnet, dass** es mehrere parallele Führungen für eine gleichzeitige Entnahme von Molekülen an verschiedenen Stellen des Organs umfasst.

## Claims

1. Surgical instrument (1) for sampling molecules in an organ which can be accessed directly or via a surgical portal, comprising:
- a device (5) for capturing said molecules,
- a stylet (4) supporting said capture device (5) capable of entering the organ,
- a guide (3) for inserting the stylet (4) into the body as far as the organ,
**characterized in that** the distal end of the guide (3) is provided with a damping block (6) intended to come to bear upon the organ.

2. The instrument according to claim 1, **characterized in that** the distal end of the stylet (4) is also provided with a damping block (6') arranged to ensure sealing with respect to the guide (3).

3. The instrument according to one of claims 1 or 2, **characterized in that** the damping block (6, 6') is made in a biocompatible, non-protein polymer material.

4. The instrument according to one of claims 1 to 3, **characterized in that** the damping block (6, 6') has a coefficient of friction in relation to the organ higher than that of steel.

5. The instrument according to one of claims 1 to 4, **characterized in that** the capture device (5) is arranged in a housing (40) of the distal portion of the stylet (4), so that only its active side is exposed.

6. The instrument according to one of claims 1 to 5, **characterized in that** the capture device (5) is adapted to capture proteins or any other biomolecule.

7. The instrument according to one of claims 1 to 6, **characterized in that** the stylet (4) and/or the guide (3) is provided with a neuro-navigational sensor.

8. The instrument according to one of claims 1 to 7, **characterized in that** it comprises an adjustable abutment (7) to control the depth of insertion of the stylet (4) into the organ.

9. The instrument according to one of claims 1 to 8 **characterized in that** it comprises several parallel guides for simultaneous sampling of molecules at different points
of the organ.
